(19) Europäisches Patentamt
European Patent Office
Office européen des brevets

(11) Publication number: **0 093 580**
**B1**

(12) **EUROPEAN PATENT SPECIFICATION**

(45) Date of publication of patent specification: **10.12.86**

(21) Application number: **83302397.1**

(22) Date of filing: **27.04.83**

(51) Int. Cl.⁴: **C 08 F 14/24,** C 08 F 8/20, C 07 C 17/28, C 07 C 17/20, C 07 C 19/08

(54) **Chlorotrifluoroethylene telomerization process.**

(30) Priority: **03.05.82 US 374561**

(43) Date of publication of application:
**09.11.83 Bulletin 83/45**

(45) Publication of the grant of the patent:
**10.12.86 Bulletin 86/50**

(84) Designated Contracting States:
**DE FR GB IT**

(56) References cited:
**GB-A- 761 053**
**GB-A- 927 403**
**GB-A-1 242 177**
**US-A-3 843 734**

**INDUSTRIAL AND ENGINEERING CHEMISTRY, vol. 39, 1947, Washington, W.T. MILLER et al. "Low polymers of chlorotrifluoroethylene", pages 333-337**

(73) Proprietor: **OCCIDENTAL CHEMICAL CORPORATION**
**P.O. Box 189**
**Niagara Falls New York 14302 (US)**

(72) Inventor: **Saran, Mohan S.**
**135 Gregory Place**
**Grand Island New York 14072 (US)**

(74) Representative: **Myerscough, Philip Boyd et al**
**J.A.Kemp & Co. 14, South Square Gray's Inn**
**London, WC1R 5EU (GB)**

**EP 0 093 580 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an improved process for preparing telomers of chlorotrifluoroethylene, hereinafter designated as "CTFE". CTFE telomers are saturated low molecular weight polymers, typically of general formula

$$CCl_3(CF_2CClF)_nCl,$$

where n, the molecular number (the number of repeating units in the telomer chain) is 1 to 20. Fluorination of the telomer results in replacement of one or more chlorine atoms in the terminal groups(s) with fluorine and gives products which are inherently non-flammable, thermally stable, and are particularly suitable for use as hydraulic fluids and high temperature lubricants. Such fluorination can be achieved using a variety of fluorinating agents. For example, GB—A—712 184 and GB—A—761 053 disclose the fluorination of CTFE oils using both chlorine trifluoride and cobalt trifluoride. The use of hydrofluoric acid as a fluorinating agent for CTFE oils is also disclosed in US—A—2 636 908, while US—A—2 886 607 illustrates the use of antimony trifluoride and antimony pentachloride as fluorinating agents.

Various methods of preparing CTFE telomers are known in the prior art and have been practiced commercially for many years. An article by William T. Miller, Jr. et al in *Industrial and Engineering Chemistry*, pages 333—337 (1947), entitled "Low Polymers of Chlorotrifluoroethylene", describes a process for producing low molecular weight polymers of CTFE by carrying out the polymerizing in a solution of chloroform using benzoyl peroxide as a polymerization promoter. Other solvents disclosed in the reference as being useful for this purpose include carbon tetrachloride and tetrachloroethylene. The solution is heated in a pressure vessel for 1-3/4 hours at 100°C, and the unreacted CTFE monomer and chloroform are removed by distillation, leaving a "crude" telomer of general formula

$$CHCl_2(CF_2CClF)_nCl,$$

which can be further heated and distilled to yield products ranging from a light oil to a semi-solid wax or grease.

US—A—2 793 201, issued May 21, 1957, discloses improved promoters for polymerizing CTFE monomers to produce low molecular weight polymers. Specific promoters disclosed in the reference include various peroxides such as bis-(trichloroacetyl) peroxide and bis-(perchloroacrylyl) peroxide. The use of such promoters produces a more stable polymer by eliminating the amount of reactive hydrogen present in the polymer.

Another process which has been developed for producing low molecular weight CTFE polymers is described in US—A—2 788 375, issued April 9, 1957. This process comprises reacting CTFE with a saturated organic bromo compound, such as bromotrichloromethane, in the presence of actinic light in a de-oxygenated system to obtained saturated bromopolychlorofluoro compounds containing one or more CTFE units per molecule. These saturated bromopolychlorofluoro compounds can then be converted to corresponding polychlorofluoro compounds by treatment with chlorine, and subsequently fluorinated using, for example, cobalt trifluoride or chlorine trifluoride, in combination with antimony catalysts, to yield more highly fluorinated products.

A more recent development in this field is described in a series of articles by Y. Pietrasanta et al entitled "Telomerization by Redox Catalysis" appearing in the *European Polymer Journal*, Vol. 12 (1976) pages 219—233. This technology involves the reaction of a chlorinated telogen, such as carbon tetrachloride, with CTFE in the presence of benzoin and a suitable redox catalyst, such as ferric chloride hexahydrate ($FeCl_3 \cdot 6H_2O$). The telomerization reaction is suitably carried out in acetonitrile which is a common solvent for the reactants and catalysts. The telomerization reaction can be illustrated as follows:

$$CCl_4 + nCF_2{=}CFCl \xrightarrow[\text{Benzoin}]{FeCl_3} CCl_3(CF_2CFCl)_nCl \quad (1)$$

The redox method has the advantage of directly preparing a high yield of low molecular weight product without the necessity of cracking or fractionating a higher molecular weight polymer.

It is known that benzoin acts as a reducing agent for $Fe^{+3}$ ions in solution and that, in the absence of benzoin, product yield falls to less than 5%. See *European Polymer Journal, supra.* However, there are certain disadvantages associated with the use of benzoin in the redox telomerization process which could effectively render uneconomical any commercial scale process based on this technology. For example, benzoin must be removed from the crude telomer prior to fluorination or the fluorinated product will contain unsaturation as determined by the $KMnO_4$ test and, as a result, will not meet product specifications. Removal of the benzoin from the crude telomer requires the use of an absorption column, such as a column of activated alumina, which not only removes benzoin but retains a significant amount of telomer which must be reclaimed using, for example, solvent extraction. Thus, use of an activated alumina column is a costly and time-consuming procedure.

The invention provides an effective process for preparing CTFE telomers on a commercial scale without the inherent inefficiencies of prior art processes. The process provides CTFE telomers which can be directly fluorinated after removal of solvent and unreacted materials to yield a variety of stabilized products.

In accordance with the present invention, there is provided a process for preparing telomers of

chlorotrifluoroethylene comprising reacting chlorotrifluoroethylene with carbon tetrachloride in the presence of a catalytic amount of $FeCl_3$ and at least one reductant selected from iron, nickel, cobalt, vanadium, molybdenum, chromium and their alloys, the reaction being conducted in a common solvent for the reactants and catalysts. The preferred solvent is acetonitrile. The telomerization reaction is preferably conducted at a pressure of from 1.03 to 2.07 Mpa (150 p.s.i. to 300 p.s.i.) and a temperature of from 90°C to 150°C.

The crude telomer prepared by this process can be fluorinated directly after removal of solvent and unreacted materials, for example by evaporation using conventional fluorination techniques, preferably at a temperature of from 120°C to 200°C. The product is a stabilized telomer which has fluorine-substituted end groups and acceptable levels of product unsaturation.

The telomerization process of the present invention can be illustrated as follows:

$$CCl_4 + nCF_2{=}CFCl \xrightarrow[M]{FeI_3} CCl_3(CF_2CFCl)_nCl \qquad (2)$$

wherein M is at least one metal or alloy selected from iron, nickel, cobalt, vanadium, molybdenum, chromium and their alloys.

The molecular weight distribution of the telomer is dependent on several factors, including the relative concentration of the $Fe^{+3}$ ions in the solution, the rate of reduction of $Fe^{+3}$ ions to $Fe^{+2}$ ions, and the relative concentration of the reactants in the solution. By carefully controlling these conditions during the telomerization reaction, it is possible to produce a telomer having a relatively precise molecular weight distribution in the desired range, i.e. corresponding to a molecular number of from 1 to 20.

Although the concentration of the reactants in accordance with the present invention can vary depending on the reaction conditions and degree of telomerization desired, it has been found that particularly useful products can be obtained by maintaining a ratio of CTFE/$CCl_4$ in the range of from 1.5/1 to 0.5/1 during the telomerization reaction. Similarly, it is also desirable, although not essential, to maintain the concentration of $FeCl_3$, at from 0.05% to 10% more preferably at from 0.1% to 5%, by weight of CTFE.

During the telomerization reaction, and inordinate oxidation of $Fe^{+3}$ form occurs as a result of side reactions other than (2). In accordance with the invention it has been found necessary to introduce a reductant into the reaction mixture to reduce the $Fe^{+3}$ ions at a moderate rate. The use of benzoin as a reducing agent, although effective, has the disadvantage of requiring an additional unit operation, i.e. adsorption using, for instance, a column of activated alumina, for removal of the benzoin prior to fluorination. The present invention obviates the use of benzoin by utilizing, as a reductant, at least one metal selected from iron, nickel, cobalt, vanadium, molybdenum and chromium, or alloys of these metals, such as Hastelloy C (Hastelloy is a registered trademark of the Union Carbide Corporation). The reductant may be physically present in the reaction mixture in a variety of forms, such as a powder, particles of various sizes, wires, plates, or as a cladding material on the internal surface of the reactor vessel. The preferred form is a finely divided powder which is uniformly dispersed in the reaction vessel by means of a mechanical agitation, such as in a stirred reactor. The reductant is desirably present in the reaction mixture in the range of from 0.05% to 10%, more preferably from 0.1 to 5%, by weight of CTFE.

The telomerization reaction is preferably conducted in a stirred reactor under elevated temperature and pressure conditions, with temperatures generally ranging from 90°C to 150°C, and pressures generally in the range of from 0.34 to 2.75 MPa (50 p.s.i. to 400 p.s.i.).

The crude CTFE telomer which is prepared according to the procedure described above, can then be stripped of solvent and unreacted monomer and fluorinated directly with a suitable fluorinating agent to produce stabilized telomers. Fluorinating agents which can be employed for this purpose include cobalt trifluoride and chlorine trifluoride, among others.

The following Examples illustrate the preparation of crude CTFE telomers using various metal reductants according to the present invention.

Example 1

A one-liter glass-lined stainless steel autoclave was dried, purged with $N_2$ and charged with 166 ml. of $CCl_4$, 88 ml. of $CH_3CN$, 193 grams of CTFE, and 1.1 grams of $FeCl_3$. Nickel wire 0,9 m (36 inches) long 0,16 cm, (0.0628 inches) in diameter, and 46 $cm^2$ (7.1 square inches) surface area) was wound around the stirring shaft of the autoclave dipping in the reaction mixture. The autoclave was heated to a reaction temperature of 115°C and a pressure of 1,37 MPa (198 p.s.i.). After 1 hour, the pressure of the reaction mixture dropped to 1,24 MPa (180 p.s.i.) 70 grams of CTFE was added to increase the pressre to 1,40 MPa (200 p.s.i.). After 1-3/4 hours an additional 64 grams of CTFE was added to increase the pressure to 1,45 MPa (210 p.s.i.). The reaction was stopped after 4 hours, the temperature was allowed to fall to ambient and unreacted CTFE was removed. The contents of the autoclave were emptied and stripped to yield 249 grams of product.

The crude reaction product was fluorinated to stabilize the end groups. After filtration, the product was colorless and showed no unsaturation when tested using the $KMnO_4$ oxidation test. The $KMnO_4$ test is a standardized technique for determining unsaturation of telomers of this type, and generally comprises adding 0.06 ml. of 1% aqueous $KMnO_4$ to a solution of 1 gram of telomer dissolved in 10 ml. of acetone. If

the solution remains pink in color for at least 15 minutes, the telomer contains satisfactory limits of unsaturation.

Example 2

Following the procedure of Example 1, an autoclave lined with Hastelloy C was dried, purged with $N_2$, and charged with 48 ml. of $CCl_4$, 26 ml. of $CH_3CN$, 64.8 grams of CTFE and 0.99 grams of $FeCl_3$. The reaction mixture was heated to a temperature of 110°C and stirred for 2 hours. During this procedure, the pressure varied from about 0,4 MPa (60 p.s.i.) to about 0,8 MPa (115 p.s.i.). The temperature was then allowed to fall to ambient and unreacted CTFE was removed. The contents of the reactor were emptied and stripped to yield a product weighing 28.5 grams.

Example 3

Following the procedure of Example 1, an autoclave lined with Teflon was dried, purged with $N_2$, and charged with 247.3 grams of $CCl_4$, 65.2 grams of $CH_3CN$, 169.5 grams of CTFE, 2.0 grams of $FeCl_3$ and 0.7 grams of iron filings. The reaction mixture was heated to a temperature of 110°C and stirred for 2 hours. During this procedure, the pressure varied from 1,19 MPa (172 p.s.i.) to 1,37 MPa (199 p.s.i.). The temperature was then allowed to fall to ambient and unreacted CTFE was removed. The contents of the reactor were emptied and stripped to yield 148.0 grams of product.

Example 4

Following the procedure of Example 1, an autoclave lined with Teflon was dried, purged with $N_2$, and charged with 245 grams of $CCl_4$, 65 grams $CH_3CN$, 189 grams of CTFE, 2.0 grams of $FeCl_3$, and 0.65 grams of chromium powder. The reaction mixture was heated to a temperature of about 110°C and stirred for 2 hours. During this procedure, the pressure varied from 1,22 MPa (177 p.s.i.) to 1,37 MPa (199 p.s.i.). The temperature was then allowed to fall to ambient and unreacted CTFE was removed. The contents of the reactor were emptied and stripped to yield 173 grams of product.

**Claims**

1. A process for preparing telomers of chlorotrifluoroethylene comprising reacting chlorotrifluoroethylene with carbon tetrachloride in the presence of a redox catalyst, characterized in that the reaction is carried out in the presence of a catalytic amount of $FeCl_3$ and at least one reductant selected from iron, cobalt, vanadium, nickel, molybdenum, chromium and their alloys, said reaction being conducted in a common solvent for the reactants and catalysts.

2. A process according to claim 1 wherein the common solvent is acetonitrile.

3. A process according to claim 2 wherein the $FeCl_3$ is present in the reaction mixture in an amount of from 0.05% to 10% by weight of chlorotrifluoroethylene.

4. A process according to claim 3 wherein the reductant is present in the reaction mixture in an amount of from 0.05% to 10% by weight of chlorotrifluoroethylene.

5. A process according to any one of the preceding claims wherein the telomerization reaction is conducted at a pressure of from 150 p.s.i. to 300 p.s.i. (1.03 to 2.07 MPa).

6. A process according to any one of the preceding claims wherein the telomerization reaction is conducted at a temperature of from 90°C to 150°C.

7. A process for preparing a stabilized telomer of chlorotrifluoroethylene, which comprises the steps of:

a) reacting chlorotrifluoroethylene with carbon tetrachloride in accordance with a process as claimed in any one of claims 1 to 6,

b) removing solvent and unreacted materials from the telomer produced in step a), and

c) directly fluorinating the telomer from step b) to stabilize the telomer.

8. A process according to claim 7 wherein the solvent and unreacted materials are removed from the telomer by evaporation.

9. A process according to claim 7 or 8 wherein the telomer is fluorinated with chlorine trifluoride.

10. A process according to claim 7, 8 or 9 wherein the fluorination reaction in step (c) is conducted at a temperature of from 120°C to 200°C.

**Patentansprüche**

1. Verfahren zur Herstellung von Telomeren des Chlortrifluorethylens, wobei Chlortrifluorethylen mit Kohlenstofftetachlorid in Gegenwart eines Redoxkatalysators umgesetzt wird, dadurch gekennzeichnet, daß die Reaktion in Gegenwart einer katalytischen Menge an $FeCl_3$ und mindestens eines Reduktionsmittels aus der Gruppe von Eisen, Kobalt, Vanadin, Nickel, Molybdän, Chrom und deren Legierungen durchgeführt wird, wobei die Reaktion in einem gemeinsamen Lösungsmittel für Reaktanten und Katalysatoren durchgeführt wird.

2. Verfahren nach Anspruch 1, worin das gemeinsame Lösungsmittel Acetonitril ist.

3. Verfahren nach Anspruch 2, worin das $FeCl_3$ in dem Reaktionsgemisch in einer Menge von 0,05 bis 10 Gew.-% Chlortrifluorethylen vorhanden ist.

4. Verfahren nach Anspruch 3, worin das Reduktionsmittel in dem Reacktionsgemisch in einer Menge von 0,05 bis 10 Gew.-% Chlortrifluorethylen vorhanden ist.

5. Verfahren nach einem der voranstehenden Ansprüche, worin die Telomerisierungsreaktion bei einem Druck von 1,03 bis 2,07 MPa (150 psi bis 300 psi) durchgeführt wird.

6. Verfahren nach einem der voranstehenden Ansprüche, worin die Telomerisierungsreaktion

bei einer Temperatur von 90 bis 150°C durchgeführt wird.

7. Verfahren zur Herstellung eines stabilisierten Telomers des Chlortrifluorethylens, wobei die folgenden Verfahrensschritte durchgeführt werden:

a) Umsetzung des Chlortrifluorethylens mit Kohlenstofftetrachlorid nach einem Verfahren gemäß der Beanspruchung in einem der Ansprüche 1 bis 6,

b) Entfernung des Lösungsmittels und des nicht umgesetzten Materials von dem in Stufe a) hergestellten Telomer, und

c) direkte Fluorierung des Telomers aus Stufe b) zur Stabilisierung des Telomers.

8. Verfahren nach Anspruch 7, worin das Lösungsmittel und die nicht umgesetzten Materialien vom Telomer durch Verdampfung abgetrennt werden.

9. Verfahren nach Anspruch 7 oder 8, worin das Telomer mit Chlortrifluorid fluoriert wird.

10. Verfahren nach Anspruch 7, 8 oder 9, worin die Fluorierungsreacktion in Stufe c) bei einer Temperatur von 120 bis 200°C durchgeführt wird.

**Revendications**

1. Procédé pour préparer des télomères de chlorotrifluoroéthylène consistant à faire réagir le chlorotrifluoroéthylène avec du tétrachlorure de carbone en la présence d'un catalyseur redox, caractérisé en ce que la réaction est conduite en présence d'une quantité catalytique de $FeCl_3$ et d'au moins un réducteur choisi parmi le fer, le cobalt, le vanadium, le nickel, le molybdène, le chrome et leurs alliages, cette réaction étant conduite dans un solvant commun aux réactifs et aux catalyseurs.

2. Procédé selon la revendication 1, dans lequel le solvant commun est l'acétonitrile.

3. Procédé selon la revendication 2, dans lequel la quantité de $FeCl_3$ dans le mélange de réaction est comprise entre 0,05 et 10% en poids du chlorotrifluoroéthylène.

4. Procédé selon la revendication 3, dans lequel la quantité de réducteur présent dans le mélange de réaction est comprise entre 0,05 et 10% en poids du chlorotrifluoroéthylène.

5. Procédé selon l'une quelconque des revendications précédentes, dans lequel la réaction de télomérisation est conduite sous une pression comprise entre 1,03 et 2,07 MPa.

6. Procédé selon l'une quelconque des revendications précédentes, dans lequel la réaction de télomérisation est conduite à une température comprise entre 90°C et 150°C.

7. Procédé pour préparer un télomère stabilisé de chlorotrifluoroéthylène, qui comporte les stades suivants:

a) faire réagir le chlorotrifluoroéthylène avec du tétrachlorure de carbone selon un procédé revendiqué dans l'une quelconque des revendications 1 à 6,

b) enlever le solvant et les produits n'ayant pas réagi du télomère produit au stade (a), et

c) fluorer directement le télomère provenant du stade (b) pour stabiliser le télomère.

8. Procédé selon la revendication 7, dans lequel le solvant et les produits n'ayant pas réagi sont enlevés du télomère par évaporation.

9. Pocédé selon la revendication 7 ou la revendication 8, dans lequel le télomère est fluoré par du trifluorure de chlore.

10. Procédé selon la revendication 7, 8 ou 9, dans lequel la réaction de fluoration au stade (c) est conduite à une température comprise entre 120°C et 200°C.